Europäisches Patentamt

European Patent Office

Office européen des brevets

(11) Publication number: **0 469 610 A1**

# EUROPEAN PATENT APPLICATION

(21) Application number: **91112959.1**

(22) Date of filing: **01.08.91**

(51) Int. Cl.5: **C12Q 1/68**, C12Q 1/70

(30) Priority: **02.08.90 JP 206290/90**

(43) Date of publication of application:
**05.02.92 Bulletin 92/06**

(84) Designated Contracting States:
**AT BE CH DE DK ES FR GB GR IT LI LU NL SE**

(71) Applicant: **SHIONOGI SEIYAKU KABUSHIKI KAISHA trading under the name of SHIONOGI & CO. LTD.**
**1-8, Doshomachi 3-chome Chuo-ku Osaka 541(JP)**

(72) Inventor: **Igarashi, Hisanaga**
**3-1-1-801, Furuichi, Joto-ku**
**Osaka-shi, Osaka(JP)**
Inventor: **Aono, Yuko**
**5F-808, Misawa-cho**
**Ibaraki-shi, Osaka(JP)**
Inventor: **Orita, Satoshi**
**1-8-211, Nakasato-cho, Kita-ku**
**Kobe-shi, Hyogo(JP)**
Inventor: **Sato, Akihiko**
**2-3-21, Minamiminohara**
**Ibaraki-shi, Osaka(JP)**

(74) Representative: **Vossius & Partner**
**Siebertstrasse 4 P.O. Box 86 07 67**
**W-8000 München 86(DE)**

(54) **Improved two-step PCR method.**

(57) The present invention relates to a two-step polymerase chain reaction method with high sensitivity and high specificity, wherein the primer concentration in the first-step PCR is very low.

EP 0 469 610 A1

The present invention relates to a two-step polymerase chain reaction method (a two-step PCR method). More particularly, it relates to a two-step PCR method with high sensitivity and high specificity, wherein the primer concentration in the first-step PCR is very low.

The PCR (Polymerase Chain Reaction) refers to a method for the amplification of a particular DNA fragment utilizing the fact that primers are required for DNA polymerase. The advantage of this PCR method is that an infinitesimal amount of DNAs can be amplified up to a required amount of DNAs. This is based on the principle that with the use of two primers (oligonucleotides) designed for a target portion of the base sequence, in the presence of four deoxyribonucleoside triphosphates [dNTP (dATP, dCTP, dGTP and dTTP)], the second strand in the portion interposed between the two primers is synthesized by DNA polymerase with the first strand of the double-stranded DNA as a template. In an actual method, particular DNAs are amplified by repeating the following processes as one cycle up to the number of cycles required under the optimum conditions of temperature and duration (Saiki, R. K. et al., Science 230, 1350-1354, 1985): a change of DNAs into a single-stranded form by thermal denaturation (denaturation process); annealing of primers to combine with template DNAs (annealing process); and synthesis of complementary DNAs by DNA polymerase (extension process). In practice, the repetition of the foregoing processes in about 30 cycles makes possible $10^5$ to $10^6$-fold amplification of a DNA fragment. Moreover, recent development of thermostable DNA polymerase makes possible the automation of PCR, thereby expanding its application field (Saiki, R. K. et al., Science 239, 487-489, 1988; and Chien, A. et al., J. Bacteriology 127, 1550-1557, 1976). In the field of microbiology, the PCR method has also found wide applications as a high-sensitive detection method for bacteria, viruses, and other microorganisms.

However, the PCR method commonly used at the present time has many disadvantages. It seems possible to make an infinite amplification in principle, but it is not so easy in actual cases. In many cases, if the cycle number of amplification is increased, then the amplification of a non-specific DNA fragment becomes observed in addition to the amplification of a particular DNA fragment. Accordingly, the cycle number of amplification which can be actually used is limited. In many cases, even if the cycle number at the limit is used, it is not possible to attain a DNA amplification up to an amount enough for easy detection. For this reason, it is quite usual to increase the detection sensitivity by the use of radio isotope (RI) or the like to compensate for this disadvantage. However, such a method requires troublesome operations with peculiar equipments because of the use of RI. In addition, there is a disadvantage that it takes a few days or more to obtain the results.

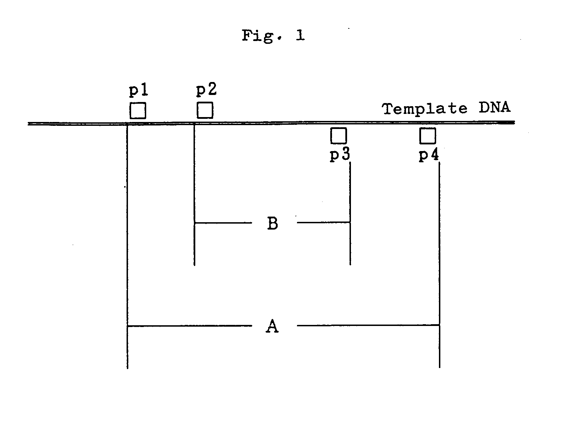

Recently, some attempts to overcome the above-mentioned disadvantages have been reported. In particular, as a PCR method using a very small amount of template DNAs, a nested PCR method has been devised (Mullis, K. B. et al., Method Enzymol. 155, 335-350, 1987). Further, this method has been applied to the detection of hepatitis B virus (HBV) DNA and human immunodeficiency virus (HIV) DNA as a PCR-PCR method (Kaneko, S. et al., J. Clin. Microbiology 27, 1930-1933, 1989; and Simmonds, P. et al., J. Virology 64, 864-872, 1990). The principle of this method is that PCR is conducted twice, and its important feature is primer sets used in the first- and second-step PCRs. That is to say, the primer set in the second step will take an inside position close to the primer set in the first step, with relation to the position of a target DNA (see Figure 1). Therefore, it results in an amplification by the use of amplified DNAs obtained in the first-step PCR as template DNAs in the second-step PCR. Thus, the above-mentioned disadvantages are nearly overcome. It is possible to ensure the degree of amplification sufficiently to detect its products by a simple detection means and it is also possible to obtain theoretically a higher specificity than by conventional methods.

Thus, the PCR method has always been improved so as to obtain a higher specificity by a simpler process. As a result of the study performed by the present inventors, it was found that the above-mentioned method involving a two-times simple repetition of PCR was not satisfying. That is to say, the amount of target DNAs in a specimen is usually unknown, and when the amount of target DNAs (copy number) is significantly varied between the specimens in the range of from a very small amount to a large amount, this method using the same conditions both in the first- and second-step PCRs has a disadvantage of poor specificity.

Thus, the technical problem underlying the present invention was to develop a PCR method capable of readily detecting a very small amount of DNAs with higher specificity and higher sensitivity. That is to say, the objective of the present invention is to provide a PCR method capable of attaining an easy, quick, safe, economic detection of a very small amount of DNA which have been undetectable hitherto, and further having a higher specificity than that of conventional methods.

The solution to this technical problem is achieved by providing the embodiments characterized in the claims.

That is to say, the present invention relates to:

(1) A two-step PCR method wherein PCR is conducted twice, comprising the improvement that the primer concentration in the first-step PCR is in the range of from 5 to 25 nM;

(2) A two-step PCR method wherein the primer concentration in the first-step PCR is in the range of from 10 to 25 nM;

(3) A two-step PCR method wherein a template DNA is human T-cell leukemia virus type-1 (HTLV-1) DNA, and wherein the primer concentration in the first-step PCR is in the range of from 5 to 25 nM, and more preferably 25 nM;

(4) A two-step PCR method wherein a template DNA is human T-cell leukemia virus type-2 (HTLV-2) DNA, and wherein the primer concentration in the first-step PCR is in the range of from 5 to 25 nM, and more preferably 10 nM;

(5) A two-step PCR method wherein a template DNA is HIV-1 DNA, and wherein the primer concentration in the first-step PCR is in the range of from 5 to 25 nM, and more preferably from 10 to 25 nM; and

(6) A two-step PCR method, wherein the PCR method is a nested-primer PCR method.

The two-step PCR method of the present invention has a high specificity and high precision which have not been achieved by any conventional PCR methods. According to the two-step PCR method of the present invention, the presence of one or more DNA molecules of interest makes possible the macroscopic detection by the naked eye. Moreover, it is possible to perform the detection easily, quickly, safely, and economically, without need of blotting, hybridization, and labeling by RI and biotin or the like.

Thus, the two-step PCR method is applicable not only to the DNA diagnosis of HTLV-1, HIV and any other viruses, but also to the DNA diagnosis of other microorganisms. Further, it can be extensively used and useful for all the methods employing a PCR method, such as a gene diagnosis utilized in general genetics and forensic medicine. In particular, it will exhibit its high ability in the PCR examination for template DNAs contained in a mixture of small copy-number specimens and large copy-number specimens.

As described above, the two-step PCR method of the present invention is of great use, as compared with the known PCR methods.

The figures show:

Figure 1: Relative position of primers in the two-step PCR of the present invention.

The primer concentration in the first-step PCR of the present invention is settled as 1/40 to 1/100 of such a concentration usually used as 1 $\mu$M, and is a necessary and sufficient quantity to achieve the above-mentioned objective. In the case of any other concentrations, non-specific bands will appear or macroscopic detection by the naked eye will become impossible in the detection by electrophoresis and subsequent ethidium bromide staining. It is desirable that the primer concentration in the first-step PCR should be determined, case by case, within the above-mentioned range, depending upon the kind of template DNA or the precision of the experimental apparatus.

Three kinds of template DNAs used in the present invention are HTLV-1 infected cell strain DNA (100 ng/$\mu$l), HTLV-2 clonal plasmid DNA ($1.2 \times 10^6$ copies/pg/$\mu$l), and HIV-1 infected cell strain DNA (100 ng/$\mu$l). These template DNAs are used in a dilution of $10^{-1}$ to $10^{-7 \text{ to } -9}$ with a control cell strain DNA which is negative for all the three kinds of template DNAs. Besides these, there are applicable DNAs of any other viruses such as Epstein-Barr virus (EBV), human papilloma virus (HPV), and hepatitis B virus (HBV).

As the primer set, four species (p1, p2, p3 and p4; See Fig. 1.) of 15 - 40 mer oligonucleotides or preferably about 30 mer oligonucleotides are synthesized by an automatic synthesizer for each template DNA. This primer set is used in such a manner that primers p1 and p4 are used in the first-step PCR and primers p2 and p3 are used in the second-step PCR, wherein primers p1 and p2 may be identical to each other and primers p3 and p4 may be identical to each other.

The conditions of PCR are as described below. In the case of the first-step PCR, the denaturation process is conducted at 92 to 96$^\circ$C for 0.5 to 2 min., and preferably at 94$^\circ$C for 1 min.; the annealing process is conducted at 50 to 72$^\circ$C for 0.5 to 2 min., and preferably at 60 to 63$^\circ$C for 1 min.; the extension process is conducted at 70 to 74$^\circ$C for 1 to 3 min., and preferably at 72$^\circ$C for 2 min.; and these processes as one cycle are repeated in 20 to 30 cycles and preferably in 25 cycles.

In the case of the second-step PCR, the primer concentration is an ordinary one, for example, 0.5 to 1 $\mu$M; the denaturation process is conducted at 92 to 96$^\circ$C for 0.5 to 2 min., and preferably at 94$^\circ$C for 0.5 to 1 min.; the annealing process is conducted at 50 to 72$^\circ$C for 0.5 to 2 min., and preferably at 65 to 68$^\circ$C for 1 min.; the extension process is conducted at 72 to 74$^\circ$C for 1 to 3 min., and preferably at 72$^\circ$C for 1.5 to 2 min.; and these processes as one cycle are repeated in 25 to 35 cycles and preferably in 30 cycles.

The thermostable DNA polymerase is used in 2 to 3 units and preferably in 2.5 units per assay for each of the first and second steps.

As the method for the detection of amplified DNA fragments, electrophoresis is conducted on a mixed gel of agarose and Nusieve (trade name, Takara Shuzo) together with molecular markers, followed by ethidium bromide staining and ultraviolet irradiation for detection.

The examples illustrate the invention.

Example-1 (Preparation of template DNAs)

Each of the various DNAs described below was used as a template DNA. The template DNA in Item (d) was used as a diluent to make the DNA concentration constant.

(a) High-molecular DNA of the HTLV-1 infected cells (TL-SU, (Sugamura, K. et al., Int. J. Cancer 34, 221-228, 1984)): 100 ng/$\mu$l;

(b) HTLV-2 clonal plasmid DNA (pH 6 - B 3.5) (Shimotohno, K. et al., Proc. Natl. Acad. Sci. USA 82, 3101-3105, 1985): 1.2$\times$ 10$^6$ copies/pg/$\mu$l;

(c) High-molecular DNA of the HIV-1 infected cells (HTLV-IIIb infected Molt 4 cells, (Matsuyama, T. et al., J. Virology 63, 2504-2509, 1989)): 100 ng/$\mu$l;

(d) High-molecular DNA of the control cell line which is negative for all of HTLV-1, HTLV-2 and HIV-1 (human glioblastoma cell line; A172, (Igarashi, H. et al., Oncogene 1, 79-85, 1987)): 100 ng/$\mu$l.

Example-2 (Preparation of primers)

As a primer set, each of the four species (p1, p2, p3 and p4) of about 30-mer oligonucleotides was synthesized by an automatic synthesizer (Cyclone, Biosearch Co.). The base sequence, position, and length of the respective primer sets to be used for each template DNA are as follows.

4

HTLV-1 (Seiki, M. et al., Proc. Natl. Acad. Sci. USA 80, 3618-3622, 1983):

    pIX1 : 5'CCCACTTCCCAGGGTTTGGACAGAGTCTTC3' (SEQ ID NO. 1)

           nt; 7324-7353, 30mer

    pIX2 : 5'CGGATACCCAGTCTACGTGTTTGGAGACTGT3' (SEQ ID NO. 2)

           nt; 7358-7388, 31mer

    pIX3 : 5'GAGCCGATAACGCGTCCATCGATGGGGTCC3' (SEQ ID NO. 3)

           nt; 7487-7516, 30mer

    pIX4 : 5'GGGGAAGGAGGGGAGTCGAGGGATAAGGAA3' (SEQ ID NO. 4)

           nt; 7527-7556, 30mer

HTLV-2 (Shimotohno, K. et al., Proc. Natl. Acad. Sci. USA 82, 3101-3105, 1985):

    pIIE1: 5'CACACATTTTCCACTAGCCCAGCAGAGCCG3' (SEQ ID NO. 5)

           nt; 5215-5244, 30mer

    pIIE2: 5'CTCACGATTGGTATCTCCTCCTACCACTCC3' (SEQ ID NO. 6)

           nt; 5252-5281, 30mer

    pIIE3: 5'TTAGGGCAGGGGGGGTGTAGTCGTTGGTCC3' (SEQ ID NO. 7)

           nt; 5344-5373, 30mer

    pIIE4: 5'GAAGGCGAGTAGTACCCTAGGCCCTGTCTG3' (SEQ ID NO. 8)

           nt; 5446-5475, 30mer

HIV-1 (Ratner, L. et al. Nature 313, 277-284, 1985):

    pHIX1: 5'GGGTGTCGACATAGCAGAATAGGCGTTACT3' (SEQ ID NO. 9)

           nt; 5781-5810, 30mer

    pHIX2: 5'ATGGAGCCAGTAGATCCTAGACTAGAGCCC3' (SEQ ID NO. 10)

           nt; 5830-5859, 30mer

    pHIX3: 5'CGCTTCTTCCTGCCATAGGAGATGCCTAAG3' (SEQ ID NO. 11)

           nt; 5955-5984, 30mer

    pHIX4: 5'AGGAGGTCTTCGTCGCTGTCTCCGCTTCTT3' (SEQ ID NO. 12)

           nt; 5977-6006, 30mer

Example-3 (Two-step PCR method)

For PCR, GeneAmp Kit (PERKIN ELMER CETUS; Takara Shuzo) was used, and the reaction was conducted using PERKIN ELMER CETUS DNA Thermal Cycler. Each of the template DNA in Items (a), (b) and (c) of Example-1 was used as a specimen in a ten fold dilution (i.e., $10^{-1}$ to $10^{-7 \text{ to } -9}$) with the template DNA in Item (d). Thus, a series of model specimens containing from a large number of target DNA copies ($10^5$ copies) to a small number of target DNA copies (1 copy or less) are obtained. In addition, the template DNA in Item (d) was used as a negative control specimen.

The respective primer sets prepared in Example-2 (Table 1) are used as shown in Figure 1; a combination of p1 and p4 is used for amplifying the DNA of size A, while a combination of p2 and p3 is used for amplifying the DNA of size B. From the primers p1 to p4, p1 and p2 are sense primers, while p3 and p4 are antisense primers.

## Table 1

| Template DNA | Primer set | | | |
| --- | --- | --- | --- | --- |
| | First-step | | Second-step | |
| | p1 | p4 | p2 | p3 |
| HTLV-1 | pIX1 | pIX4 | pIX2 | pIX3 |
| HTLV-2 | pIIE1 | pIIE4 | pIIE2 | pIIE3 |
| HIV-1 | pHIX1 | pHIX4 | pHIX2 | pHIX3 |

(1) First-step PCR

In this PCR step, 5 $\mu$l of each of the step-wise-diluted template DNAs (corresponding to 0.5 $\mu$g of DNA), 10 $\mu$l of a 10 $\times$ reaction buffer [500 mM KCl, 100 mM Tris-HCl (pH 8.3), 15 mM $MgCl_2$, 0.1% (w/v) gelatin], 16 $\mu$l of a dNTP mixture [1.25 mM dNTP (dATP, dCTP, dGTP and dTTP)], 1000 - 0.1 nM of each of the primers (p1 and p4), and 2.5 units/assay thermostable DNA polymerase were mixed together, and the total volume of the solution was brought to 100 $\mu$l. The denaturation process, annealing process, extension process, and total number of cycles for the respective template DNAs are listed in Table 2 below. These conditions were respectively determined as a result of various examinations.

Table 2

| Template DNA | Denaturation | Annealing | Extension | Cycle No. |
| --- | --- | --- | --- | --- |
| HTLV-1 | 94$^\circ$C 1 min. | 63$^\circ$C, 1 min. | 72$^\circ$C, 2 min. | 25 |
| HTLV-2 | 94$^\circ$C 1 min. | 63$^\circ$C, 1 min. | 72$^\circ$C, 2 min. | 25 |
| HIV-1 | 94$^\circ$C 1 min. | 60$^\circ$C, 1 min. | 72$^\circ$C, 2 min. | 25 |

(2) Second-step PCR

In this PCR step, 10 $\mu$l of each of the first-step PCR products as a template DNA, 10 $\mu$l of the same 10 $\times$ reaction buffer as used in the first-step, 16 $\mu$l of a dNTP mixture [1.25 mM dNTP (dATP, dCTP, dGTP and dTTP)], 1.0 $\mu$M of each of the primers (p2 and p3), and 2.5 units/assay thermostable DNA polymerase were mixed together, and the total volume of the solution was brought to 100 $\mu$l. The denaturation process, annealing process, extension process, and total number of cycles for the respective template DNAs are as listed in Table 3 below. These conditions were respectively determined as a result of various examinations.

Table 3

| Template DNA | Denaturation | Annealing | Extension | Cycle No. |
|---|---|---|---|---|
| HTLV-1 | 94°C, 0.5 min. | 68°C, 1 min. | 72°C, 1.5 min. | 30 |
| HTLV-2 | 94°C, 0.5 min. | 65°C, 1 min. | 72°C, 1.5 min. | 30 |
| HIV-1 | 94°C, 1 min. | 65°C, 1 min. | 72°C, 2 min. | 30 |

(3) Detection of amplified gene fragments by gel electrophoresis and ethidium bromide staining

First, 1/10 volume, 10 $\mu$l, of each of the products obtained in the second-step PCR was subjected to electrophoresis on a mixed gel of 2.0% Nusieve (trade name, Takara Shuzo Co.) and 1.0% agarose together with DNA molecular weight markers. Then, an ethidium bromide stained image was obtained by ultraviolet irradiation, and photographed with a Polaroid film, followed by the judgement of detection. The theoretical target sizes (of the second-step PCR products) for the respective template DNAs are shown in Table 4 below.

| Table 4 | |
|---|---|
| Template DNA | Target size (Second-step PCR products) |
| HTLV-1 | 159 bps |
| HTLV-2 | 122 bps |
| HIV-1 | 155 bps |

(4) Results

i) The following results are those obtained in the two-step PCR by using five serial concentrations of 1000, 100, 10, 1, and 0.1 nM for the primer concentration in the first-step PCR, and by using HTLV-1 DNA as a template DNA.

| Primer concentration (nM) | Detection limit (Dilution of template DNA) | Non-specific band |
|---|---|---|
| 1000 | $10^{-6}$ | appeared |
| 100 | $10^{-6}$ | appeared |
| 10 | $10^{-6}$ | none |
| 1 | $10^{-3}$ | none |
| 0.1 | $10^{-2}$ | none |

These results showed that the primer concentration capable of attaining a satisfactory detection sensitivity and specificity is 10 nM.

ii) Since it was found from the results in Item i) that the primer concentration in the first-step PCR is preferably 10 nM, a more detailed examination was made with respect to the four concentrations of 50, 25, 10, and 5 nM around this concentration of 10 nM. The results are shown in the table below. In this examination, the three template DNAs in Items (a), (b) and (c) of Example-1 were used for the purpose of confirming the possibility that the applied field of the present method will be extended.

7

| Template DNA | Primer concentration (nM) | Detection limit (Dilution of template DNA) | Appearance of non-specific bands (Dilution of template DNA) |
|---|---|---|---|
| HTLV-1 | 50 | $10^{-6}$ to $-7$ | $10^{-1}$ to $10^{-2}$ (slight) |
|  | 25 | $10^{-6}$ to $-7$ | ——— |
|  | 10 | $10^{-6}$ | ——— |
|  | 5 | $10^{-5}$ | ——— |
| HTLV-2 | 50 | $10^{-7}$ | $10^{-1}$ to $10^{-2}$ |
|  | 25 | $10^{-6}$ to $-7$ | $10^{-1}$ to $10^{-4}$ |
|  | 10 | $10^{-6}$ to $-7$ | ——— |
|  | 5 | $10^{-5}$ to $-6$ | ——— |
| HIV-1 | 50 | $10^{-6}$ | $10^{-1}$ to $10^{-2}$ (slight) |
|  | 25 | $10^{-5}$ | ——— |
|  | 10 | $10^{-5}$ | ——— |
|  | 5 | $10^{-4}$ to $-5$ | ——— |

These results will lead to the following:

① In the case of HTLV-1, it was found that the primer concentration capable of attaining a satisfactory detection sensitivity and specificity is in the range of from 5 to 25 nM with the optimum primer concentration being 25 nM which makes possible the specific detection up to a dilution of $10^{-7}$. On the basis of the facts that the TL-Su cell used herein contains 10 to 20 copies of the HTLV-1 genome per cell, and that the amount of DNAs contained in $10^5$ human cells corresponds to about 1 $\mu$g, it is found by calculation that there are $10^{-2}$ TL-Su cells (approximately 0.1 to 0.2 copies of the HTLV-1 genome) in 5 $\mu$l of a $10^{-7}$ dilution (0.5 $\mu$g). In the repeated experiments using the same template DNA, detection up to a dilution of $10^{-6}$ was frequently achieved, which indicates that 1 to 2 or more copies of the HTLV-1 genome can be detected by the use of a dilution of $10^{-6}$.

② In the case of HTLV-2, it was found that the primer concentration capable of attaining a satisfactory detection sensitivity and specificity is in the range of from 5 to 25 nM with the optimum primer concentration being 10 nM which makes possible the specific detection up to a dilution of $10^{-7}$. On the basis of the fact that the HTLV-2 clonal plasmid DNA used herein contains the HTLV-2 genome corresponding to $1.2 \times 10^{-6}$ copies/pg/$\mu$l, it is found by calculation that there are approximately 0.6 copies of the HTLV-2 genome in 5 $\mu$l of a $10^{-7}$ dilution. In the repeated experiments using the same template DNA, detection only to a dilution of $10^{-6}$ was sometimes observed, which indicates that 0.6 to 6.0 or more copies of the HTLV-2 genome can be detected by the use of a dilution of $10^{-6 \text{ to } -7}$.

③ In the case of HIV-1, it was found that the primer concentration capable of attaining a satisfactory detection sensitivity and specificity is in the range of from 5 to 25 nM with the optimum primer concentration being in the range of from 10 to 25 nM which makes possible the specific detection up to a dilution of $10^{-5}$. Although the precise number of the HIV-1 genome contained in each HIV-1 infected cell is unknown, it is found by calculation that there are 0.5 HIV-1 infected cells in 5 $\mu$l (0.5 $\mu$g) of a $10^{-5}$ dilution on the basis of the fact that the amount of DNAs contained in $10^5$ human cells corresponds to about 1 $\mu$g. Therefore, it can be said that the detection limit is 0.5 infected cells in this case.

Putting these results together, it seems that the primer concentration used in the first-step PCR by the two-step PCR method of the present invention is preferably in the range of from 5 to 25 nM, more preferably, the optimum primer concentration being in the range of from 10 to 25 nM. However, when the copy number of the template DNA in the subjects is presumed to be extremely small (1 to 10 copies), the concentration of 25 nM is recommended to be used. In conducting an experiment, it is preferred that the practical primer concentration is determined within this concentration range on the basis of the experimental precision in conformity with the actual cases.

In addition, from the fact that copy numbers below a decimal point can never be considered, and from the interpretation that a fluctuation of the maximum detection limit in the experiments (i.e., when using a dilution of $10^{-7}$ in the above-mentioned cases (1) and (2), there were some bands appearing and disappearing, depending upon the experiments) may be caused by a dilution error and a sampling error, it can be concluded that the two-step PCR method of the present invention has a sensitivity sufficient to detect one copy of the template DNA. Moreover, it was shown that the specificity of the two-step PCR method of the present invention is satisfactory for practical applications, because non-specific bands were not detected in the subjects containing a template DNA in the copy number of from $10^5$ down to 1.

**Claims**

1.  A two-step PCR method wherein PCR is conducted twice and the primer concentration in the first-step PCR is in the range of from 5 to 25 nM.

2.  The two-step PCR method according to claim 1, wherein said primer concentration is in the range of from 10 to 25 nM.

3.  The two-step PCR method according to claim 1 or 2, wherein a template DNA is HTLV-1 DNA, HTLV-2 DNA, or HIV-1 DNA.

4.  The two-step PCR method according to any one of claims 1 to 3, wherein said PCR is nested-primer PCR.

Fig. 1

European
Patent Office

EUROPEAN SEARCH
REPORT

Application Number

EP 91 11 2959

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | PROCEEDINGS OF THE NATIONAL ACADEMY OF SCIEN-CESvol. 86, April 1989, WASHINGTON,D.C.,USA pages 2423 - 2427; D.J.KEMP ET AL.: 'Colorimetric detection of specific DNA segments amplified by polymerase chain reactions' * abstract * * * page 2423, right column, line 49 - page 2424, left column, line 23 * EP 91112959030* * page 2426, left column, line 25 - page 2427, right column, line 8 * * | 1-4 | C 12 Q 1/68 C 12 Q 1/70 |
| | – – – | | |
| A | H.A. ERLICH,EDITOR 'PCR Technology:principles and applications for DNA amplification' October 1989 , STOCKTON PRESS , NEW YORK,USA * page 7 - page 16 * Esp. p. 15 "Factors affecting specificity" * | 1,4 | |
| | – – – | | |
| A | EP-A-0 357 011   (ABBOTT LABORATORIES) * page 5, line 15 - line 31 * * * page 7, line 10 - line 45 * * | 1,4 | |
| | – – – | | |
| P,A | WO-A-9 015 881   (CIS BIO INTERNATIONAL) * page 6, line 3 - page 14, line 9 * * * page 16, line 15 - line 36 * * | 1-4 | |
| | – – – – – | | TECHNICAL FIELDS SEARCHED (Int. Cl.5) |
| | | | C 12 Q |

The present search report has been drawn up for all claims

| Place of search | Date of completion of search | Examiner |
|---|---|---|
| The Hague | 04 November 91 | LUZZATTO E.R.P.G.A. |


## SEQUENCE LISTING

INFORMATION FOR SEQ ID NO:1:

    (i) SEQUENCE CHARACTERISTICS:

        (A)LENGTH: 30 base pairs

        (B)TYPE: nucleic acid

        (C)STRANDEDNESS: single

        (D)TOPOLOGY: linear

    (ii) MOLECULE TYPE: other nucleic acid, synthesized DNA

    (iv) ANTI-SENSE: no

    (xi) SEQUENCE DESCRIPTION: SEQ ID NO:1:


CCCACTTCCC AGGGTTTGGA CAGAGTCTTC          30


INFORMATION FOR SEQ ID NO:2:

    (i) SEQUENCE CHARACTERISTICS:

        (A)LENGTH: 31 base pairs

        (B)TYPE: nucleic acid

        (C)STRANDEDNESS: single

        (D)TOPOLOGY: linear

    (ii) MOLECULE TYPE: other nucleic acid, synthesized DNA

    (iv) ANTI-SENSE: no

    (xi) SEQUENCE DESCRIPTION: SEQ ID NO:2:


CGGATACCCA GTCTACGTGT TTGGAGACTG T          31


INFORMATION FOR SEQ ID NO:3:

    (i) SEQUENCE CHARACTERISTICS:

        (A)LENGTH: 30 base pairs

(B)TYPE: nucleic acid

(C)STRANDEDNESS: single

(D)TOPOLOGY: linear

(ii) MOLECULE TYPE: other nucleic acid, synthesized DNA

(iv) ANTI-SENSE: yes

(xi) SEQUENCE DESCRIPTION: SEQ ID NO:3:


GAGCCGATAA CGCGTCCATC GATGGGGTCC                                    30



INFORMATION FOR SEQ ID NO:4:

    (i) SEQUENCE CHARACTERISTICS:

        (A)LENGTH: 30 base pairs

        (B)TYPE: nucleic acid

        (C)STRANDEDNESS: single

        (D)TOPOLOGY: linear

(ii) MOLECULE TYPE: other nucleic acid, synthesized DNA

(iv) ANTI-SENSE: yes

(xi) SEQUENCE DESCRIPTION: SEQ ID NO:4:


GGGGAAGGAG GGGAGTCGAG GGATAAGGAA                                    30



INFORMATION FOR SEQ ID NO:5:

    (i) SEQUENCE CHARACTERISTICS:

        (A)LENGTH: 30 base pairs

        (B)TYPE: nucleic acid

        (C)STRANDEDNESS: single

        (D)TOPOLOGY: linear

(ii) MOLECULE TYPE: other nucleic acid, synthesized DNA

(iv) ANTI-SENSE: no

(xi) SEQUENCE DESCRIPTION: SEQ ID NO:5:


CACACATTTT CCACTAGCCC AGCAGAGCCG                                            30


INFORMATION FOR SEQ ID NO:6:

      (i) SEQUENCE CHARACTERISTICS:

            (A)LENGTH: 30 base pairs

            (B)TYPE: nucleic acid

            (C)STRANDEDNESS: single

            (D)TOPOLOGY: linear

     (ii) MOLECULE TYPE: other nucleic acid, synthesized DNA

     (iv) ANTI-SENSE: no

     (xi) SEQUENCE DESCRIPTION: SEQ ID NO:6:


CTCACGATTG GTATCTCCTC CTACCACTCC                                            30


INFORMATION FOR SEQ ID NO:7:

      (i) SEQUENCE CHARACTERISTICS:

            (A)LENGTH: 30 base pairs

            (B)TYPE: nucleic acid

            (C)STRANDEDNESS: single

            (D)TOPOLOGY: linear

     (ii) MOLECULE TYPE: other nucleic acid, synthesized DNA

     (iv) ANTI-SENSE: yes

     (xi) SEQUENCE DESCRIPTION: SEQ ID NO:7:


TTAGGGCAGG GGGGGTGTAG TCGTTGGTCC                                            30

INFORMATION FOR SEQ ID NO:8:

    (i) SEQUENCE CHARACTERISTICS:

        (A)LENGTH: 30 base pairs

        (B)TYPE: nucleic acid

        (C)STRANDEDNESS: single

        (D)TOPOLOGY: linear

    (ii) MOLECULE TYPE: other nucleic acid, synthesized DNA

    (iv) ANTI-SENSE: yes

    (xi) SEQUENCE DESCRIPTION: SEQ ID NO:8:


GAAGGCGAGT AGTACCCTAG GCCCTGTCTG           30


INFORMATION FOR SEQ ID NO:9:

    (i) SEQUENCE CHARACTERISTICS:

        (A)LENGTH: 30 base pairs

        (B)TYPE: nucleic acid

        (C)STRANDEDNESS: single

        (D)TOPOLOGY: linear

    (ii) MOLECULE TYPE: other nucleic acid, synthesized DNA

    (iv) ANTI-SENSE: no

    (xi) SEQUENCE DESCRIPTION: SEQ ID NO:9:


GGGTGTCGAC ATAGCAGAAT AGGCGTTACT           30


INFORMATION FOR SEQ ID NO:10:

    (i) SEQUENCE CHARACTERISTICS:

(A)LENGTH: 30 base pairs

(B)TYPE: nucleic acid

(C)STRANDEDNESS: single

(D)TOPOLOGY: linear

(ii) MOLECULE TYPE: other nucleic acid, synthesized DNA

(iv) ANTI-SENSE: no

(xi) SEQUENCE DESCRIPTION: SEQ ID NO:10:


ATGGAGCCAG TAGATCCTAG ACTAGAGCCC                          30


INFORMATION FOR SEQ ID NO:11:

    (i) SEQUENCE CHARACTERISTICS:

        (A)LENGTH: 30 base pairs

        (B)TYPE: nucleic acid

        (C)STRANDEDNESS: single

        (D)TOPOLOGY: linear

    (ii) MOLECULE TYPE: other nucleic acid, synthesized DNA

    (iv) ANTI-SENSE: yes

    (xi) SEQUENCE DESCRIPTION: SEQ ID NO:11:


CGCTTCTTCC TGCCATAGGA GATGCCTAAG                          30


INFORMATION FOR SEQ ID NO:12:

    (i) SEQUENCE CHARACTERISTICS:

        (A)LENGTH: 30 base pairs

        (B)TYPE: nucleic acid

        (C)STRANDEDNESS: single

        (D)TOPOLOGY: linear

(ii) MOLECULE TYPE: other nucleic acid, synthesized DNA

(iv) ANTI-SENSE: yes

(xi) SEQUENCE DESCRIPTION: SEQ ID NO:12:


AGGAGGTCTT CGTCGCTGTC TCCGCTTCTT                    30